# EUROPEAN PATENT SPECIFICATION

(11) **EP 1 945 022 B1**
(45) Date of publication and mention of the grant of the patent: **05.09.2018**
(21) Application number: 06804587.1
(22) Date of filing: 20.10.2006
(51) Int. Cl.: C12N 15/63, C12N 15/64, C12N 15/82, C12N 15/00, A01H 5/02, A01H 5/08, A01H 5/10

(54) **POLYNUCLEOTIDES, DNA CONSTRUCTS AND METHODS FOR THE ALTERATION OF PLANT CELLULOSE CONTENT**
POLYNUKLEOTIDE, DNA-KONSTRUKTE UND VERFAHREN ZUR VERÄNDERUNG DES CELLULOSEGEHALTS EINER PFLANZE
POLYNUCLEOTIDES, STRUCTURES D'ADN ET PROCEDES POUR LA MODIFICATION DE LA TENEUR EN CELLULOSE DE PLANTES

(30) Priority: 21.10.2005 US 728743 P
(43) Date of publication of application: 23.07.2008
(73) Proprietor: Fibria Celulose S/A, Sao Paulo / SP (BR)
(72) Inventor: PAPES, Fabio, 13069-380 Campinas/SP (BR); ARRUDA, Paulo, 13069-380 Campinas/SP (BR)
(74) Representative: D Young & Co LLP
(86) International application number: PCT/BR2006/000225
(87) International publication number: WO 2007/045063

(56) References cited:
- WO-A-98/03637
- US-A1- 2002 116 736
- US-A1- 2002 124 281
- US-B1- 6 682 918
- KONISHI TERUKO ET AL: "Evidence that sucrose loaded into the phloem of a poplar leaf is used directly by sucrose synthase associated with various beta-glucan synthases in the stem." PLANT PHYSIOLOGY (ROCKVILLE), vol. 134, no. 3, March 2004 (2004-03), pages 1146-1152, XP002557652 ISSN: 0032-0889
- NAKAI TOMONORI ET AL: "Enhancement of cellulose production by expression of sucrose synthase in Acetobacter xylinum" PROCEEDINGS OF THE NATIONAL ACADEMY OF SCIENCES OF THE UNITED STATES OF AMERICA, vol. 96, no. 1, 5 January 1999 (1999-01-05), pages 14-18, XP002557653 ISSN: 0027-8424
- AMOR YEHUDIT ET AL: "A membrane-associated form of sucrose synthase and its potential role in synthesis of cellulose and callose in plants" PROCEEDINGS OF THE NATIONAL ACADEMY OF SCIENCES OF USA, NATIONAL ACADEMY OF SCIENCE, WASHINGTON, DC, US, vol. 92, no. 20, 1 January 1995 (1995-01-01), pages 9353-9357, XP002435928 ISSN: 0027-8424

## Description

### RELATED APPLICATION

This application claims priority of application Serial No. U.S. 60/728,743 filed on October 21, 2005.

### FIELD OF INVENTION

The present invention relates to the field of molecular biology and the regulation of protein synthesis through the introduction of foreign genes into plant cells, preferably into their genomes. More specifically, the method relates to the modification of cellulose content in a plant cell by the introduction of a foreign gene encoding an active sucrose synthase enzyme.

### BACKGROUND

Sucrose synthase (EC 2.4.1.13) is an enzyme that catalyzes the breakdown of sucrose to UDP-glucose and fructose. In plants, sucrose synthase is found in sink tissues such as tubers, seeds, fruits, meristems and wood. Subsequent reactions in storage tissues utilize UDP-glucose to generate starch, while in other tissues UDP-glucose and fructose may be accumulated or further converted into other compounds, such as cellulose. Multiple sucrose synthase isoforms have been identified in plants, most of them typically displaying a tissue-specific pattern of expression. Huang et al., Biosci. Biotech. Biochem, 60:233-239 (1996); Chourey et al., Mol. Gen. Genet. 203:251-255 (1986). In particular, several sucrose synthase isoforms are found predominantly or exclusively in tissues where biomass growth through massive cell division and cell wall deposition take place, such as developing secondary xylem and root nodules. Rouhier & Usuda, Plant Cell Physiol. 42:583-593 (2001); Komina et al., Plant Physiol. 129: 1664-1673 (2002).

Plants regulate sucrose synthase at both transcriptional and post-translational levels, including feedback inhibition by glucose/fructose and phosphorylation. Maize sucrose synthase has been shown to be reversibly phosphorylated at Ser-15 residue, which
is conserved among all plant sequences examined to date. Hardin et al., Plant Physiol. 134: 1427-1438 (2004).

In cyanobacteria, such as *Anabaena* sp., sucrose synthase seems to play a role in nitrogen fixation in specialized structures called heterocysts. Schilling & Ehrnsperger, Z. Naturforsch 40c: 776-779 (1985). It has been suggested that, in these organisms, sucrose synthase is responsible for sucrose synthesis, while its function in plants is sucrose breakdown. Salerno & Curatti, Trends Plant Sci. 8: 63-69 (2003).

Cellulose is one of the major components of plant cell walls, providing mechanical strength and rigidity. Normally, oven-dry wood contains 30 to 50% cellulose and 20 to 30% hemicellulose, see Higuchi et al., BIOCHEMISTRY AND MOLECULAR BIOLOGY OF WOOD, Springer Verlag (1997), although these numbers usually change when the tree is subject to environmental changes. For example, wood under compression stress usually exhibits a marked decrease in cellulose content, while tension wood has more cellulose. Timmell, COMPRESSION WOOD IN GYMNOSPERMS, Springer Verlag (1986).

Cellulose is a polymer of beta-1,4-linked glucose residues synthesized by one or more cellulose synthase enzymes which catalyze the assembly of UDP-glucose units into cellulose microfibrils in protein complexes known as rosettes at the plasma membrane. Delmer & Amor, Plant Cell 7: 987-1000 (1995). In higher plants, the degree of glucan polymerization varies between primary and secondary cell walls, with secondary walls producing cellulose microfibrils of higher molecular weight. Brown et al., Trends Plant Sci. 1: 149-156 (1996). Cell walls in tensioned wood also possess elevated cellulose content and increased polymerization and crystallinity degrees. Such variations in the degree of polymerization in cell walls are thought to depend on which cellulose synthase isoforms are involved in cellulose synthesis. Haigler & Blanton, Proc. Natl Acad. Sci. USA 93: 12082-12085 (1996).

On the other hand, little is known on how cellulose synthase complexes work and whether these protein complexes are able to couple cellulose synthesis with sucrose breakdown, which is thought to provide UDP-glucose units for cellulose synthesis in all plant cells through the action of sucrose synthases. Delmer, Annu. Rev. Plant Physiol. Plant Mol. Biol. 50: 245-276 (1999).

Konishi et al., Plant Physiol. 134: 1146-1152 (2004) report on overexpressing mung bean sucrose synthase in 25 independent poplar lines. While all of the lines displayed high levels of sucrose synthase in the leaves, none displayed increased cellulose deposition. The authors speculate that this result might pertain because plants possess regulatory mechanisms that control sugar transport from source to sink, particularly when source tissues contain high sucrose synthase levels. Alternatively, Konishi et al. conjecture that the absence of increased cellulose deposition in their transgenic lines might be associated with the use of a constitutive promoter, which could have led to inefficient sucrose partition between source and sink.

### SUMMARY OF THE INVENTION

In one aspect, the invention provides a transgenic plant comprising a non-plant sucrose synthase nucleic acid molecule, wherein said plant has altered cellulose content compared with a non-transgenic plant lacking said molecule. In one embodiment, the transgenic plant is obtained by transforming a plant with an expression vector comprising said non-plant sucrose synthase nucleic acid molecule under the control of a promoter capable of functioning in a plant. In another embodiment, the nucleic acid molecule is (a) a nucleotide sequence comprising SEQ ID NO.: 2; or (b) a nucleotide sequence of (a) modified such that the modification does not result in the loss of sucrose synthase enzyme activity compared with a non-transgenic plant lacking said nucleotide sequence.

In one embodiment, the transgenic plant has increased cellulose content. The transgenic plant may have decreased lignin content. In one embodiment, the transgenic plant is a dicotyledon or a monocotyledon. In another embodiment, the transgenic plant is a gymnospenn. In a further embodiment, the plant is a woody tree. In a still further embodiment, the woody tree is a *Eucalyptus, Populus,* or *Pinus* plant. The transgenic plant may be selected from the group consisting of a leaf, a stem, a flower, an ovary, a fruit, a seed, and a callus.

In another aspect, the invention provides a method for producing a transgenic plant with increased cellulose content as compared to a non-transgenic plant, comprising (i) transforming a plant cell with a non-plant sucrose synthase sequence under the control of a promoter capable of functioning in a plant, (ii) culturing said transformed
plant cell under conditions that promote growth of a plant, and (iii) selecting a transgenic plant that exhibits increased cellulose content.

In one embodiment, the nucleotide sequence is a sucrose synthase gene comprising: (a) a nucleotide sequence comprising SEQ ID NO.: 2; or (b) a nucleotide sequence of (a) modified such that the modification does not result in the loss of sucrose synthase enzyme activity compared with a non-transgenic plant lacking said nucleotide sequence. In one embodiment, the transgenic plant is a dicotyledon or a monocotyledon. In another embodiment, the transgenic plant is a gymnosperm.

Described herein is an isolated polynucleotide sequence comprising a nucleic acid sequence encoding a polypeptide that is capable of increasing sucrose synthase and cellulose levels in a plant.

### BRIEF DESCRIPTION OF THE DRAWINGS

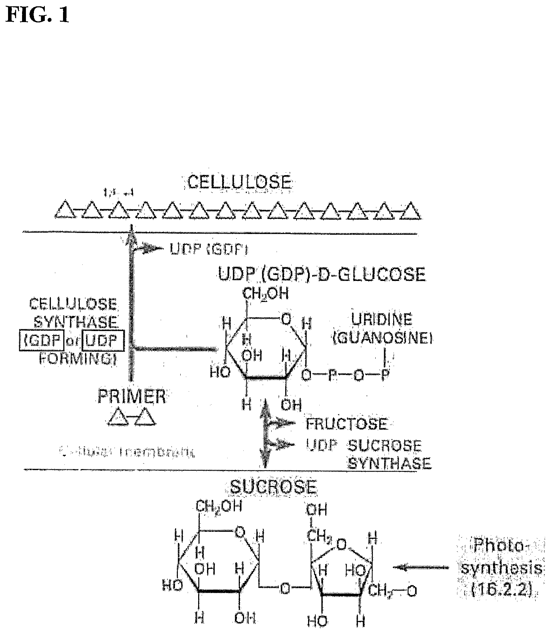

**FIG. 1** illustrates the reaction catalyzed by sucrose synthase in plants and the coupling of UDP-glucose generation with cellulose biosynthesis pathway.
**FIG. 2** schematically illustrates the plant expression plasmidial vector pALELLYX-Susy of the invention comprising a cambium/xylem preferred promoter driving the expression of a sucrose synthase nucleotide sequence of the invention.
**FIG. 3** schematically illustrates the plant expression plasmidial vector pALELLYX-Susy_{N} of the invention comprising a cambium/xylem preferred promoter driving the expression of a native sucrose synthase nucleotide sequence from *Anabaena* sp.
**FIG. 4** schematically illustrates the plant expression plasmidial vector pALELLYX-Susy_{CU} of the invention comprising a cambium/xylem preferred promoter driving the expression of codon usage-adapted nucleotide sequence coding for the *Anabaena* sp. sucrose synthase enzyme.
**FIG. 5** shows the cellulose content of several transgenic lines transformed with the plant expression plasmidial vector pALELLYX-Susy_{N} of the invention and respective control non-transgenic plant. Asterisk denotes statistically significant higher mean cellulose content values (P<0.01, t-test).
**FIG. 6** shows the cellulose content of several transgenic lines transformed with the plant expression plasmidial vector pALELLYX-Susy_{CU} of the invention and
   respective control non-transgenic plant. Asterisk denotes statistically significant higher mean cellulose content values (P<0.05, t-test).
**FIG. 7** shows the cellulose content of the three genotypes of a T1 transgenic plant transformed with the plant expression plasmidial vector pALELLYX-Susy_{CU} of the invention. Asterisk denotes statistically significant higher mean cellulose content values (P<0.05, t-test).

### DETAILED DESCRIPTION OF THE INVENTION

The present inventors recognized that non-plant sucrose synthases, such as those found in cyanobacteria, might not be regulated by a plant that was genetically engineered to express the enzyme, for reasons that include but are not limited to the absence of regulatory phosphorylation or other allosteric sites such as those usually found in plant sucrose synthases. By the same token, the inventors had the insight that a non-plant sucrose synthase, introduced into such a host plant, could affect sucrose synthesis and yet would not be subject to down-regulation by the host plant. Thus, the present inventors realized the desirability of transforming woody trees and other cellulose fiber-producing crops with a DNA construct that codes for a non-plant sucrose synthase enzyme. The heterologous enzyme would increase the conversion of glucose into UDP-glucose, which then would be used as a building block for cellulose synthesis.

Accordingly, the present invention relates to compositions comprising non-plant sucrose synthase-encoding DNA sequences and sucrose synthase polypeptides. Illustrative of nucleotide sequences that encode a sucrose synthase are SEQ ID NOs: 1 and 2.

According to an aspect of the present invention, a method is provided for modifying the content of cellulose in plant tissues, such as fiber cells of woody angiosperm xylem, tracheid cells of gymnosperm xylem, and fiber cells of cotton seeds, by controlling the activity of sucrose synthase. Pursuant to this aspect of the invention, plant cells or whole plants are transformed with a sucrose synthase coding sequence from a non-plant source, preferably an *Anabaena* sp., which sequence, when expressed in xylary fiber cells of angiosperms, xylary tracheids of gymenosperms, or fiber cells of cotton seeds, causes
increased conversion of sucrose into UDP-glucose, leading to increased UDP-glucose availability for cellulose biosynthesis.

In addition to increasing cellulose biosynthesis and deposition, the present disclosure provides a means for concurrently reducing lignin deposition. The high concentration of lignin in trees presents a significant problem for the paper industry, which must expend considerable resources to separate lignin from cellulose fiber. Accordingly, it is desirable to reduce lignin content in woody plants via genetic engineering. Hu et al., Nature Biotechnology 17: 808-812 (1999), found that suppression of lignin biosynthesis increased cellulose deposition, which suggested an inverse relationship between lignin and cellulose biosynthesis. Because the present inventors have discovered a strategy for increasing cellulose deposition through expression of a non-plant sucrose synthase, concurrent decreases in lignin deposition also may result.

All technical terms used herein are terms commonly used in biochemistry, molecular biology and agriculture, and can be understood by one of ordinary skill in the art to which this invention belongs. Those technical terms can be found in: MOLECULAR CLONING: A LABORATORY MANUAL, 3rd ed., vol. 1-3, ed. Sambrook and Russel, Cold Spring Harbor Laboratory Press, Cold Spring Harbor, N.Y., 2001; CURRENT PROTOCOLS IN MOLECULAR BIOLOGY, ed. Ausubel et al., Greene Publishing Associates and Wiley-Interscience, New York, 1988 (with periodic updates); SHORT PROTOCOLS IN MOLECULAR BIOLOGY: A COMPENDIUM OF METHODS FROM CURRENT PROTOCOLS IN MOLECULAR BIOLOGY, 5th ed., vol. 1-2, ed. Ausubel et al., John Wiley & Sons, Inc., 2002; GENOME ANALYSIS: A LABORATORY MANUAL, vol. 1-2, ed. Green et al., Cold Spring Harbor Laboratory Press, Cold Spring Harbor, N.Y., 1997. Methodology involving plant biology techniques is described herein and is described in detail in treatises such as METHODS IN PLANT MOLECULAR BIOLOGY: A LABORATORY COURSE MANUAL, ed. Maliga et al., Cold Spring Harbor Laboratory Press, Cold Spring Harbor, N.Y., 1995. Various techniques using PCR are described, e.g., in Innis et al., PCR PROTOCOLS: A GUDIE TO METHODS AND APPLICATIONS, Academic Press, San Diego, 1990 and in Dieffenbach and Dveksler, PCR PRIMER: A LABORATORY MANUAL, 2nd ed., Cold Spring Harbor Laboratory Press, Cold Spring Harbor, N.Y., 2003. PCR-primer pairs can be derived from known sequences by known techniques such as using computer programs intended for that purpose, e.g., Primer,
Version 0.5, 1991, Whitehead Institute for Biomedical Research, Cambridge, MA. Methods for chemical synthesis of nucleic acids are discussed, for example, in Beaucage and Caruthers, Tetra. Letts. 22:1859-1862 (1981), and Matteucci and Caruthers, J. Am. Chem. Soc. 103:3185 (1981).

Restriction enzyme digestions, phosphorylations, ligations and transformations were done as described in Sambrook et al., MOLECULAR CLONING: A LABORATORY MANUAL, 2nd ed. (1989), Cold Spring Harbor Laboratory Press. All reagents and materials used for the growth and maintenance of bacterial cells were obtained from Aldrich Chemicals (Milwaukee, WI), DIFCO Laboratories (Detroit, MI), Invitrogen (Gaithersburg, MD), or Sigma Chemical Company (St. Louis, MO) unless otherwise specified.

The terms "encoding" and "coding" refer to the process by which a gene, through the mechanisms of transcription and translation, provides information to a cell from which a series of amino acids can be assembled into a specific amino acid sequence to produce an active enzyme. Because of the degeneracy of the genetic code, certain base changes in DNA sequence do not change the amino acid sequence of a protein. It is therefore understood that modifications in the DNA sequence encoding sucrose synthase which do not substantially affect the functional properties of sucrose synthase enzyme are contemplated.

In this description, "expression" denotes the production of the protein product encoded by a gene. "Overexpression" refers to the production of a gene product in transgenic organisms that exceeds levels of production in normal or non-transformed organisms.

### Non-Plant Sucrose Synthase Sequences

Sucrose synthase genes have been identified in several non-plant species, exemplified by cyanobacteria (*Anabeana* sp.), proteobacteria (*N. europaea*), cholorophyta (*C. vulgaris* and *S. obliquus*), and protista (*E. gracilis*). Accordingly, the phrase "non-plant sucrose synthase" refers here to any nucleic acid, gene, polynucleotide, DNA, RNA, mRNA, or cDNA molecule that is isolated from the genome of a non-plant species that confers sucrose synthase activity.

A non-plant sucrose synthase suitable for the present invention may be obtained from a myriad of organisms that are characterized by the presence of a sucrose synthase gene that is less regulated by a host plant that contains and expresses the gene. For instance, plant sucrose synthase sequences contain phosphorylation domains that regulate gene expression, whereas many cyanobacterial sucrose synthase sequences, for instance, lack phosphorylation domains. Unlike plant sucrose synthases, non-plant sucrose synthases are believed to be less subject to negative feedback inhibition in a host plant.

For purposes of the present invention, a non-plant sucrose synthase gene preferably is isolated from a cyanobacterial species. More preferably, a non-plant sucrose synthase gene is isolated from a filamentous and heterocystic cyanobacteria. Illustrative cyanbobacterial species are *Aphanocapsa* sp., *Aphanothece* sp., *Chamaesiphon* sp., *Chroococcus* sp., *Chroogloeocystis* sp., *Crocosphaera* sp., *Cyanobacterium* sp., *Cyanobium* sp., *Cyanothece* sp., *Dactylococcopsis* sp., *Gloeocapsa* sp., *Gloeothece* sp., *Johannesbaptistia* sp., *Merismopedia* sp., *Microcystis* sp., *Rhabdoderma* sp., *Synechococcus* sp., *Synechocystis* sp., *Thermosynechococcus* sp., *Anabaena* sp., *Anabaenopsis* sp., *Aphanizomenon* sp., *Aulosira* sp., *Cyanospira* sp., *Cylindrospermopsis* sp., *Cylindrospermum* sp., *Mojavia* sp., *Nodularia* sp., *Nostoc* sp., *Raphidiopsis* sp., *Trichormus* sp., *Calothrix* sp., *Gloeotrichia* sp., *Scytonema* sp., *Scytonematopsis* sp., *Arthrospira* sp., *Geitlerinema* sp., *Halomicronema* sp., *Halospirulina* sp., *Katagnymene* sp., *Leptolyngbya* sp., *Limnothrix* sp., *Lyngbya* sp., *Microcoleus* sp., *Oscillatoria* sp., *Phormidium* sp., *Planktothricoides* sp., *Planktothrix* sp., *Plectonema* sp., *Pseudanabaena* sp., *Schizothrix* sp., *Spirulina* sp., *Symploca* sp., *Trichodesmium* sp., *Tychonema* sp., *Chroococcidiopsis* sp., *Dermocarpa* sp., *Dermocarpella* sp., *Myxosarcina* sp., *Pleurocapsa* sp., *Stanieria* sp., *Xenococcus* sp., *Prochloron* sp., *Prochlorococcus* sp., *Prochlorothrix* sp., *Capsosira* sp., *Chlorogloeopsis* sp., *Fischerella* sp., *Hapalosiphon* sp., *Mastigocladopsis* sp., *Nostochopsis* sp., *Stigonema* sp., *Symphyonema* sp., *Umezakia* sp., *Westiellopsis* sp., *Acaryochloris* sp.

For example, a sucrose synthase gene isolated from *Anabaena* sp. is a non-plant sucrose synthase and can be used to modify the cellulose content of plant tissues and/or organs, in accordance with the present invention. Preferably, the non-plant sucrose synthase is obtained from *Anabaena* sp. genomic DNA, such as the sequence set forth in SEQ ID NO: 1. SEQ ID NO.: 1 represents the sucrose synthase sequence as found in the genomic DNA of *Anabaena* sp., which is less than 55% identical to plant cDNAs encoding sucrose synthase proteins. Notably, SEQ ID NO: 1 lacks a conserved phosphorylation site near the N-terminus, which is required for protein phosphorylation in eukaryotic species.

A non-plant sucrose synthase sequence may be synthesized *ab initio* as a codon-optimized sequence. See Young and Dong, Nucleid Acids Res 32: e59 (2004). For example, SEQ ID NO: 2 provides a DNA sequence encoding a sucrose synthase enzyme in which all codons were chosen to match the average preferred codons used in plants for each amino acid. Accordingly, SEQ ID NO.: 2 is less than 55% identical to plant cDNAs encoding sucrose synthase proteins, but the protein sequence encoded is identical to the protein sequence encoded by the non-codon optimized sequence, and it therefore lacks a conserved phosphorylation site near the N-terminus too.

Additionally, the category of suitable non-plant sucrose synthase sequences includes a nucleic acid molecule comprised of a variant of SEQ ID NO: 1 or SEQ ID NO: 2, with one or more bases deleted, substituted, inserted, or added, which variant codes for a polypeptide with sucrose synthase enzyme activity. Accordingly, sequences having "base sequences with one or more bases deleted, substituted, inserted, or added" retain physiological activity even when the encoded amino acid sequence has one or more amino acids substituted, deleted, inserted, or added. Nucleotide sequences that have such modifications and that code for a sucrose synthase enzyme isoform are included within the scope of the present invention. For example, the poly A tail or 5' or 3' end nontranslation regions may be deleted, and bases may be deleted to the extent that amino acids are deleted. Bases may also be substituted, as long as no frame shift results. Bases also may be "added" to the extent that amino acids are added. It is essential, however, that any such modification does not result in the loss of sucrose synthase enzyme activity. A modified DNA in this context can be obtained by modifying the DNA base sequences of the invention so that amino acids at specific sites are substituted, deleted, inserted, or added by site-specific mutagenesis, for example. Zoller & Smith, Nucleic Acid Res. 10: 6487-6500 (1982).

A non-plant sucrose synthase sequence can be synthesized *ab initio* from the appropriate bases, for example, by using the appropriate protein sequence disclosed here as a guide to create a DNA molecule that, though different from the native DNA sequence, results in the production of a protein with the same or similar amino acid sequence. This type of synthetic DNA molecule is useful when introducing into a plant a DNA sequence, coding for a heterologous protein, that reflects different (non-plant) codon usage frequencies and, if used unmodified, can result in inefficient translation by the host plant. The DNA sequence set forth as SEQ ID NO.: 2 is such a codon usage-optimized molecule, for example.

The present disclosure further provides nucleic acid molecules comprising the nucleotide sequence of SEQ ID NOs.: 1 and 2, which encode an active sucrose synthase enzyme, wherein the enzyme has amino acid sequence that corresponds to SEQ ID NO.: 3 and wherein the protein of the invention encompasses amino acid substitutions, additions and deletions that do not alter the function of the sucrose synthase enzyme. SEQ ID NO.: 3 has less than 40% sequence homology on average to the eukaryotic proteins.

### Suitable Regulatory Elements

The disclosure provides nucleic acid molecules likely to cause altered cellulose content in a transformed plant. An important aspect of the present invention is the use of DNA constructs wherein a sucrose synthase-encoding nucleotide sequence is operably linked to one or more regulatory sequences, which drive expression of the sucrose synthase-encoding sequence in certain cell types, organs, or tissues so as to alter the cellulose content of a transformed plant without unduly affecting its normal development or physiology.

The vascular system comprises xylem and phloem tissues and are collectively referred to as "vascular tissue." Vascular system-specific promoters, such as xylem-preferred promoters, are useful for effecting expression of nucleic acid molecules within the invention, specifically in vascular tissue, especially xylem tissue. Thus, "xylem-preferred" means that the nucleic acid molecules of the current invention are more active in the xylem than in any other plant tissue. The selected promoter should cause the overexpression of the non-plant sucrose synthase, pursuant to the invention, thereby to modify the size of the xylem, to modify the chemical composition of the xylem of the host plant, or both.

Suitable promoters are illustrated by but are not limited to the xylem-preferred tubulin (TUB) gene promoter, the xylem-preferred lipid transfer protein (LTP) gene promoter, and the xylem-preferred coumarate-4-hydroxylase (C4H) gene promoter. Other suitable xylem-preferred promoters are disclosed in international patent application PCT/BR2005/000041, filed Mar. 28, 2005.

Additional regulatory elements, for use in accordance with the present invention, are described below under the "DNA Constructs" subheading.

### Plants for Genetic Engineering

The present invention comprehends the genetic manipulation of plants, especially trees and cellulose fiber-producing crops such as cotton, to enhance the activity of sucrose synthase in vascular tissues or fiber cells of seeds via introducing a non-plant sucrose synthase gene, preferably under the control of a xylem-preferred promoter. The result is enhanced cellulose synthesis and deposition.

In this description, "plant" denotes any cellulose-containing plant material that can be genetically manipulated, including but not limited to differentiated or undifferentiated plant cells, protoplasts, whole plants, plant tissues, or plant organs, or any component of a plant such as a leaf, stem, root, bud, tuber, fruit, rhizome, or the like.

Plants that can be engineered in accordance with the invention include but are not limited to trees, such as *Eucalyptus* species (*E. alba, E. albens, E. amygdalina, E. aromaphloia, E. baileyana, E. balladoniensis, E. bicostata, E. botryoides, E. brachyandra, E. brassiana, E. brevistylis, E. brockwayi, E. cainaldulensis, E. ceracea, E. cloeziana, E. coccifera, E. cordata, E. cornuta, E. corticosa, E. crebra, E. croajingolensis, E. curtisii, E. dalrympleana, E. deglupta, E. delegatensis, E. delicata, E. diversicolor, E. diversifolia, E. dives, E. dolichocarpa, E. dundasii, E. dunnii, E. elata, E. erythrocorys, E. eiythrophloia, E. eudesmoides, E. falcata*, *E. gainophylla, E. glaucina, E. globulus, E. globulus subsp. bicostata, E. globulus subsp. globulus, E. gongylocarpa, E. grandis, E. grandis x urophylla, E. guilfoylei, E. gunni, E. hallii, E. houseana, E. jacksoni, E. lansdowneana, E. latisinensis, E. leucophloia, E. leucoxylon, E. lockyeri, E. lucasii, E. maidenii, E. inarginata, E. megacarpa, E. melliodora, E. michaeliana, E. microcorys, E. microtheca, E. muelleriana, E. izitens, E. nitida, E. obliqua, E. obtusiflora, E. occidentalis, E. optima, E.*
*ovata, E. pachyphylla, E. pauciflora, E. pellita, E. perriniana, E. petiolaris, E. pilularis, E. piperita, E. platyphylla, E. polyanthemos, E. populnea, E. preissiana, E. pseudoglobulus, E. pulchella, E. radiata, E. radiata subsp. radiata, E. regnans, E. risdonii, E. robertsonii, E. rodwayi, E. rubida, E. rubiginosa, E. saligna, E. salmonophloia, E. scoparia, E. sieberi, E. spathulata, E. staeri, E. stoatei, E. tenuipes, E. tenuiramis, E. tereticornis, E. tetragona, E. tetrodonta, E. tindaliae, E. torquata, E. umbra, E. urophylla, E. vernicosa, E. viminalis, E. wandoo, E. wetarensis, E. willisii, E. willisii subsp. falciformis, E. willisii subsp. willisii, E. woodwardii*), *Populus* species (*P. alba, P. alba x P. grandidentata, P. alba x P. tremula, P. alba x P. tremula var. glandulosa, P. alba x P. tremuloides, P. balsamifera, P. balsamifera subsp. trichocarpa, P. balsamifera subsp. trichocarpa x P. deltoides, P. ciliata, P. deltoides, P. euphratica, P. euramericana, P. kitakamiensis, P. lasiocarpa, P. laurifolia, P. maximowiczii, P. maximowiczii x P. balsamifera subsp. trichocarpa, P. nigra, P. sieboldii x P. grandidentata, P. suaveolens, P. szechuanica, P. tomentosa, P. tremula, P. tremula x P. tremuloides, P. tremuloides, P. wilsonii, P. canadensis, P. yunnanensis*), Conifers such as loblolly pine (*Pinus taeda*), slash pine (*Pinus elliotii*), ponderosa pine (*Pinus ponderosa*), lodgepole pine (*Pinus contorta*), and Monterey pine (*Pinus radiata*); Douglas-fir (*Pseudotsuga menziesii*); Western hemlock (*Tsuga canadensis*); Sitka spruce (*Picea glauca*); redwood (*Sequoia sempervirens*); true firs such as silver fir (*Abies amabilis*) and balsam fir (*Abies balsamea*); and cedars such as Western red cedar (*Thuja plicata*) and Alaska yellow-cedar (*Chamaecyparis nootkatensis*).

Fibre-producing plants also are included in this context. Illustrative crops are cotton (*Gossipium* spp.), flax (*Linum usitatissimum*), stinging nettle (*Urtica dioica*), hop (*Humulus lupulus*), lime trees (*Tilia cordata, T. x. europaea* and *T. platyphyllus*), spanish broom (*Spartium junceum*), ramie (*Boehmeria nivea*), paper mulberry (*Broussonetya papyrifera*), New Zealand flax (*Phormium tenax*), dogbane (*Apocynum cannabinum*), *Iris* species (*I. douglasiana, I. macrosiphon* and *I. purdyi*), milkweeds (*Asclepia species*), pineapple, banana and others. Also contemplated are forage crops, such as alfalfa, lolium, festuca and clover.

In the present description, "transgenic plant" refers to a plant that has incorporated a DNA sequence, including but not limited to genes that are not normally present in a host plant genome, DNA sequences not normally transcribed into RNA or translated into a protein ("expressed"), or any other genes or DNA sequences that one desires to introduce into the non-transformed plant, such as genes that normally may be present in the non-transformed plant but that one desires either to genetically engineer or to have altered expression. The "transgenic plant" category includes both a primary transformant and a plant that includes a transformant in its lineage, e.g., by way of standard introgression or another breeding procedure.

It is contemplated that, in some instances, the genome of an inventive transgenic plant will have been augmented through the stable introduction of a transgene. In other instances, however, the introduced gene will replace an endogenous sequence. A preferred gene in the regard, pursuant to the present invention, is a non-plant sucrose synthase DNA sequence, particularly one obtained from the cyanobacterial species *Anabaena.*

### DNA Constructs

In accordance with one aspect of the invention, a non-plant sucrose synthase sequence is incorporated into a DNA construct that is suitable for plant transformation. Such a DNA construct can be used to modify sucrose synthase gene expression in plants, as described above.

Accordingly, DNA constructs are provided that comprise a non-plant sucrose synthase sequence, under the control of a transcriptional initiation region operative in a plant, so that the construct can generate RNA in a host plant cell. The transcriptional initiation region is part of a vascular or xylem-preferred promoter, such as any of those mentioned above.

Recombinant DNA constructs may be made using standard techniques. For example, the DNA sequence for transcription may be obtained by treating a vector containing said sequence with restriction enzymes to cut out the appropriate segment. The DNA sequence for transcription may also be generated by annealing and ligating synthetic oligonucleotides or by using synthetic oligonucleotides in a polymerase chain reaction (PCR) to give suitable restriction sites at each end. The DNA sequence then is cloned into a vector containing upstream promoter and downstream terminator sequences.

The expression vectors of the invention may also contain termination sequences, which are positioned downstream of the nucleic acid molecules of the invention, such that transcription of mRNA is terminated, and polyA sequences added. Exemplary of such terminators are the cauliflower mosaic virus (CaMV) 35S terminator and the nopaline synthase gene (Tnos) terminator. The expression vector may also contain enhancers, start codons, splicing signal sequences, and targeting sequences.

Expression vectors of the invention may also contain a selection marker by which transformed plant cells can be identified in culture. The marker may be associated with the heterologous nucleic acid molecule, i.e., the gene operably linked to a promoter. As used herein, the term "marker" refers to a gene encoding a trait or a phenotype that permits the selection of, or the screening for, a plant or plant cell containing the marker. Usually, the marker gene will encode antibiotic or herbicide resistance. This allows for selection of transformed cells from among cells that are not transformed or transfected.

Examples of suitable selectable markers include adenosine deaminase, dihydrofolate reductase, hygromycin-B-phosphotransferase, thymidne kinase, xanthine-guanine phospho-ribosyltransferase, glyphosate and glufosinate resistance and aminoglycoside 3'-O-phosphotranserase (kanamycin, neomycin and G418 resistance). These markers include resistance to G418, hygromycin, bleomycin, kanamycin, and gentamicin. The construct may also contain the selectable marker gene *Bar* that confers resistance to herbicidal phosphinothricin analogs like ammonium gluphosinate. Thompson et al., EMBO J. 9: 2519-2523 (1987). Other suitable selection markers are known as well.

Replication sequences, of bacterial or viral origin, may also be included to allow the vector to be cloned in a bacterial or phage host. Preferably, a broad host range prokaryotic origin of replication is used. A selectable marker for bacteria may be included to allow selection of bacterial cells bearing the desired construct. Suitable prokaryotic selectable markers also include resistance to antibiotics such as kanamycin or tetracycline.

Other DNA sequences encoding additional functions may also be present in the vector, as is known in the art. For instance, when *Agrobacterium* is the host, T-DNA sequences may be included to facilitate the subsequent transfer to and incorporation into plant chromosomes.

### Plant Transformation

Constructs according to the invention may be used to transform any plant cell, using a suitable transformation technique. Both monocotyledonous and dicotyledonous angiosperm or gymnosperm plant cells may be transformed in various ways known to the art. For example, see Klein et al., Biotechnology 4: 583-590 (1993); Bechtold et al., C. R. Acad. Sci. Paris 316:1194-1199 (1993); Bent et al., Mol. Gen. Genet. 204:383-396 (1986); Paszowski et al., EMBO J. 3: 2717-2722 (1984); Sagi et al., Plant Cell Rep. 13: 262-266 (1994).

*Agrobacterium* species such as *A. tumefaciens* and *A. rhizogenes* can be used, for example, in accordance with Nagel et al., Microbiol Lett 67: 325 (1990). In brief, *Agrobacterium* may be transformed with a plant expression vector via, e.g., electroporation, after which the *Agrobacterium* is introduced to plant cells via, e.g., the well known leaf-disk method. Additional methods for accomplishing this include, but arc not limited to, electroporation, particle gun bombardment, calcium phosphate precipitation, and polyethylene glycol fusion, transfer into germinating pollen grains, direct transformation (Lorz et al., Mol. Genet. 199: 179-182 (1985)), and other methods known to the art. If a selection marker, such as kanamycin resistance, is employed, it makes it easier to determine which cells have been successfully transformed.

The *Agrobacterium* transformation methods discussed above are known to be useful for transforming dicots. Additionally, de la Pena, et al., Nature 325: 274-276 (1987), Rhodes, et al., Science 240: 204-207 (1988), and Shimamato, et al., Nature 328: 274-276 (1989) have transformed cereal monocots using *Agrobacterium.* Also see Bechtold, et al., C.R. Acad. Sci. Paris 316 (1994), showing the use of vacuum infiltration for *Agrobacterium*-mediated transformation.

The presence of a protein, polypeptide, or nucleic acid molecule in a particular cell can be measured to determine if, for example, a cell has been successfully transformed or transfected. The ability to carry out such assay is well known and need not be reiterated here.

### Quantifying Cellulose and Lignin Content

Transgenic plants of the invention are characterized by increased cellulose content and preferably decreased lignin content. Increased cellulose content in the genetically engineered plant is preferably achieved via increase in sucrose synthase activity in the plant tissues wherein cellulose deposition occurs. In describing a plant of the invention, "increased cellulose content" refers to a quantitative augmentation in the amount of cellulose in the plant when compared to the amount of cellulose in a wild-type plant. A quantitative increase of cellulose can be assayed by several methods, as for example by quantification based on total sugars after acid hydrolysis of polysaccharides in stem milled wood. Chiang and Sarkanen, Wood Sci. Technol., 17: 217-226 (1983); Davis, J. Wood Chem. Technol., 18: 235-252 (1988).

The cellulose content in the engineered plant of the invention can be increased to levels of about 105% to about 200%, preferably about 110% to about 175%, even more preferably about 115% to about 150% of the cellulose content of the wild-type plant. A most preferred embodiment of the plant of the invention has a cellulose content of about 120% to about 140% of the wild-type cellulose content.

Because increased cellulose biosynthesis can reduce lignin content, transgenic plants of the invention may have increased cellulose content and reduced lignin content. See Hu et al., Nature Biotechnol. 17: 808-812 (1999). In this description, therefore, the phrases "reduced lignin content" and "decreased lignin content" connote a quantitative reduction in the amount of lignin in the plant, when compared to the amount of lignin in a wild-type plant. A quantitative reduction of lignin can be assayed by several methods, as for examples the Klason lignin assay (Kirk et al., Method in Enzymol. 161: 87-101 (1988)) or acetyl bromide assay of lignin (Iiyama et al., Wood Sci. Technol. 22: 271-280 1988)).

The lignin content in the engineered plant of the invention can be reduced to levels of about 5% to about 90%, preferably about 10% to about 75%, even more preferably about 15% to about 65% of the lignin content of the wild-type plant. A most preferred embodiment of the plant of the invention has a lignin content of about 20% to about 60% of the wild-type lignin content.

Specific examples are presented below of methods for obtaining cyanobacterial sucrose synthase enzyme genes, as well as for introducing the target gene,
via *Agrobacterium,* to produce plant transformants. They are meant to be examplary and not as limitations on the present invention.

### EXAMPLE 1. Isolation of a sucrose synthase DNA sequence from Anabaena sp. PCC 7120

### (a) Genomic DNA preparation

*Anabaena* sp. PCC 7120 (ATCC) was grown in BG-11 medium under constant cold fluorescent light with gentle agitation for one week or until the culture medium showed a green color. Cyanobacterial cells were pelleted and treated with 1% Triton X-100; 10mM Tris pH 8.0; 1mM EDTA pH 8.0 at 95°C for 30 min. The lysate was extracted twice with CHCl₃ and the resulting supernatant was used as a source of template genomic DNA for the subsequent PCR reactions.

### (b) Primer design

A DNA sequence representing the sucrose synthase gene from *Anabaena variabilis* ATCC 29413 has already been determined and deposited in the GenBank under accession number AJ292758. Based on this sequence, DNA oligomers were synthesized as primers for PCR, including either the region around the first codon ATG or around the termination codon of the main ORF encoding the sucrose synthase enzyme.

Primers were designed to amplify the entire coding region of the sucrose synthase ORF, i.e., from the ATG through the translation stop codon. The sequences of the primers are given below:
Susy_s3 Length: 27 SEQ ID NO:4
   ATCCATATGTCAGAATTGATGCAAGCG
Susy_as3 Length: 33 SEQ ID NO:5
   TCAGATCTTACCGATATTTATGCTGTTCTAATA

### (c) PCR Amplification

The genomic DNA sample obtained in (a) was used as template, and the primers designed in (b) were used for PCR. The PCR steps involved 40 cycles of 1 minute at 94°C., 1 minute at 50°C., and 2 minutes at 72°C followed by an extra step of elongation at 72°C for 7 minutes. The PCR products were isolated by gel electrophoresis on 1.0% agarose followed by ethidium bromide staining of the electrophoresed gel and detection of amplified bands on a UV transilluminator. The detected amplified band was verified and cut out of the agarose gel with a razor. The pieces of gel were transferred to 1.5 mL microtubes, and the DNA fragments were isolated and purified using a GFX PCR clean-up and gel band purification kit (Amersham). The recovered DNA fragments were subcloned to the pGEM-T cloning vector (Promega), transformed into *E. coli,* and then used to prepare plasmid DNA in the usual manner, which was then sequenced by the dideoxy method (Messing, Methods in Enzymol. 101: 20-78 (1983)), using BigDye chemistry (Applied Biosystems), to yield the DNA sequence disclosed here as SEQ ID NO.1, for use pursuant to the present invention.

### EXAMPLE 2. Synthesis of a modified codon usage-adapted sucrose synthase DNA sequence

Gene synthesis was performed according to the procedure described by Young & Dong, Nucl. Acid Res. 32: e59 (2004). Briefly, 50-mer oligonucleotides covering the entire length of the final DNA sequence are synthesized such that adjacent primers show a 10bp long overlap. To synthesize a sucrose synthase gene coding for *Anabaena* sucrose synthase enzyme, 62 primers were designed, including oligonucleotides at both ends of the sequence to insert NdeI and XbaI sites in the final PCR product. In the first PCR round, the primers are separately mixed in primer extension reactions, such that each reaction contains four adjacent primers, including two of the adjacent primers in the preceding reaction. In the second step, four groups of 8 extension reactions each are pooled and subjected to another primer extension reaction, followed by a third PCR reaction with flanking primers to amplify four ∼800bp DNA fragments spanning the whole synthetic DNA sequence. These four fragments are ligated by overlap PCR in a fourth step of amplification, which is also used to include NdeI and XbaI sites at both ends of the DNA molecule.

### EXAMPLE 3. Preparation of Transgenic Nicotiana Plants

The sucrose synthase genes obtained in Examples 1 and 2 above were introduced into a plant host to produce transgenic *Nicotiana* plants.

### (a) Preparation of constructs and transformation of Agrobacterium

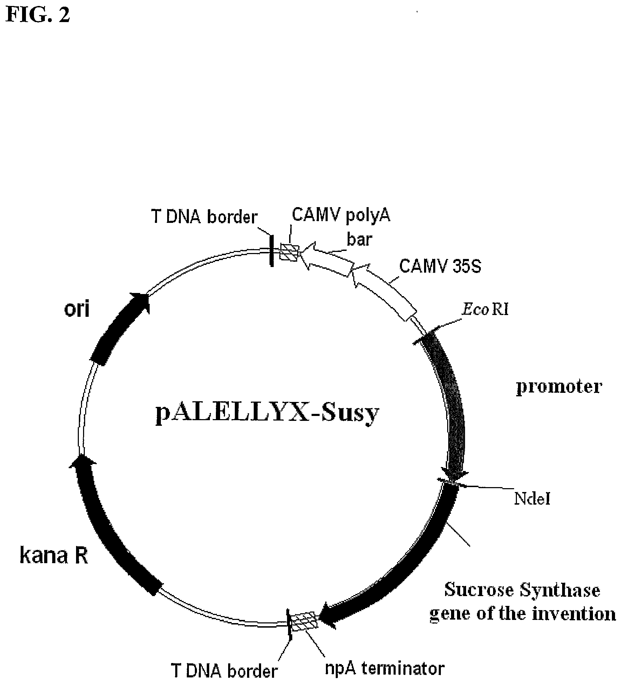

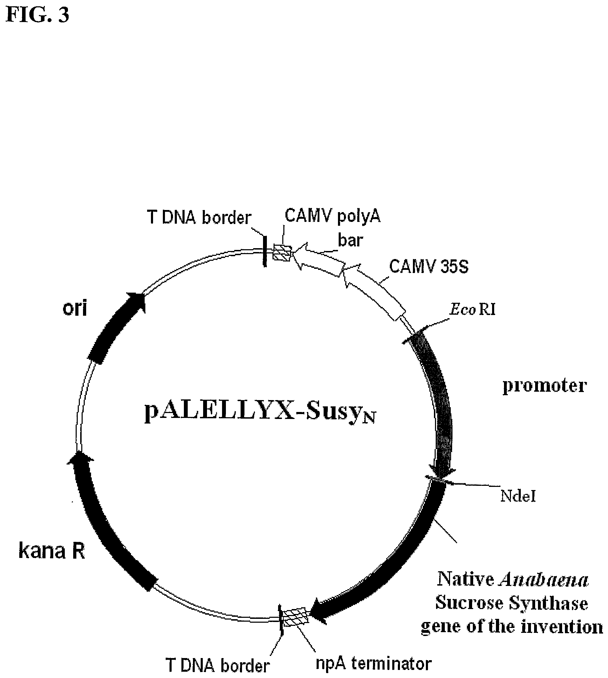

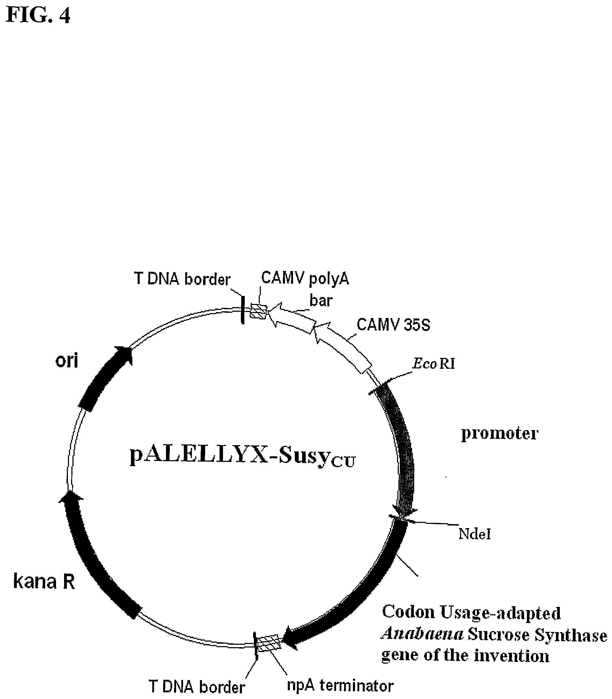

Expression constructs can be prepared by cleaving the sucrose synthase genes obtained in Examples 1 and 2 above with suitable restriction enzymes so as to include all of the open reading frame and inserting the gene into the plant transformation vector pALELLYX-Susy (FIG. 2) together with an appropriate promoter. For example, the *Anabaena* sucrose synthase gene obtained in Example 1 was cloned into the aforementioned expression vector downstream to a xylem-preferred tubulin gene (TUB) promoter from *Populus deltoides,* as set forth in international application PCT/BR2005/000041, filed March 28, 2005 (FIG. 3). Alternatively, the codon usage-adapted *Anabaena* sucrose synthase gene obtained in Example 2 was cloned into the aforementioned expression vector downstream to a xylem-preferred tubulin gene (TUB) promoter from *Populus deltoides* described above (FIG. 4). The resulting expression constructs were amplified in *E. coli,* and then transformed by freeze thawing (Nucleic Acid Res. 12, 8711 (1984)) into *Agrobacterium tumefaciens* LBA4404 strain.

### (b) Agrobacterium-mediated transformation of Nicotiana benthamiana

Transformation of *Nicotiana* sp. was accomplished using the leaf disk method of Horsch et al., Science 227: 1229 (1985), using a DNA contruct comprising the *Anabaena* native sucrose synthase gene or the synthetic codon usage-adapted sucrose synthase gene obtained in (a) operably linked to the TUB promoter of a xylem-preferred gene. The transformants were selected on Murashige and Skoog medium (Sigma, St. Louis, MO) containing 100 milligrams/liter of BASTA herbicide and 500 mg/L carbenicillin (Sigma). The transformed tobacco shoots were allowed to root on the Murashige and Skoog medium, and were subsequently transferred to soil and grown in the greenhouse.

### (c) PCR verification of foreign gene insertion into the host plant genome

PCR can be used to verify the integration of the gene construct in the genome of transgenic plants. Two specific primers were synthesized for the construct and used to PCR-amplify the corresponding construct from genomic DNA of *Nicotiana* transformants. For the construct that contains the *Anabaena* sucrose synthase ORF under the control of the *Populus* xylem-preferred tubulin gene promoter, two specific primers were synthesized:
Susy_seq3 Length 20 SEQ ID NO.: 6
   CAAGAATTGCAAGAACGTTG
Susy_as3 Length: 33 SEQ ID NO: 5
   TCAGATCTTACCGATATTTATGCTGTTCTAATA

For the construct that contains the modified synthetic codon-usage adapted *Anabaena* sucrose synthase gene under the control of the *Populus* xylem-preferred tubulin gene promoter, two specific primers were synthesized:
SUSY_CU_RT1 Length 28 SEQ ID NO.: 7
   CTAGGCTCGATAGGATCAAGAACCTCAC
SUSY_CU_RT2 Length: 28 SEQ ID NO.: 8
   GATAGCCTTCTCAGAGATGATGTTCCAG

The PCR reaction mixture contained 100 ng genomic DNA of transformed plant, and 0.2 *µ*M of each primer, 100 *µ*M of each deoxyribonucleotide triphosphate, 1×PCR buffer and 2.5 Units of AmpliTaq DNA polymerase (Applied Biosystems) in a total volume of 50 *µ*L. The cycling parameters were as follows: 94°C. for 1 minute, 50°C. for 1 minute and 72°C for 3 minutes, for 40 cycles, with 5 minutes at 72°C. extension. The PCR products were electrophoresized on an 1% agarose gel.

### (d) Determination of transgene expression level in transgenic plants

Semi-quantitative RT-PCR was used to detect the accumulation of *Anabaena* sucrose synthase transcripts in stem tissue of the transgenic plants. Total RNA was isolated from stem cuts of 3-months old transgenic *Nicotiana* T1 plants using the CTAB method described by Aldrich and Cullis, Plant Mol. Biol. Report. 11:128-141 (1993).

cDNA was synthesized from 500 ng of total RNA using Superscript II RNase H- RT (Invitrogen, USA). The primers described above were used along with primers for the constitutive gene encoding glyceraldehyde-3-phosphate dehydrogenase (GAPDH) as an internal control to normalize the quantity of total RNA used in each sample. PCR was done with a 12.5-fold dilution of the first-strand cDNA under the following conditions: 94°C for 3 min and 27 cycles of 94°C for 1 min, 52 to 60°C for 45 s, and 72°C for 1 min and 30 s.

### EXAMPLE 4. Histochemical Analysis of Transgenic Plants

Briefly, stems of transgenic *Nicotiana* and control plants were sectioned and fixed in 4% paraformaldehyde for 24hs. Fixed tissues were then sectioned on a microtome (Leica RM2255) and subsequently were stained with astra blue/saffranin. The histologically stained sections were observed under a Leica DM1L inverted microscope using bright- and dark-field illumination.

### EXAMPLE 5. Increase in Cellulose Content in Transgenic Plants Over-expressing Sucrose Synthase in Vascular Tissues

The main stems of five *Nicotiana* transgenic events transformed with a construct comprising the native *Anabaena* sp. sucrose synthase gene under the control of the xylem-preferred *Populus deltoides* tubulin promoter together and of non-transgenic control plants were collected and air-dried for two weeks. Dried stems were cut in pieces and pulverized on a knife mill using a 30-mesh sieve. Stem powder samples were then subjected to chemical analyses to determine cellulose and lignin content. Briefly, cellulose and hemicellulose contents were determined based on the total sugars after acid hydrolysis of these polysaccharides in stem wood. Wood mill was vacuum-dried at 45°C and hydrolyzed with H₂SO₄. Following high-pH anion-exchange chromatography, glucan and other polysaccharides (hemicelluloses) were quantified based on hydrolysate composition. Chiang & Sarkanen, Wood Sci. Technol. 17: 217-226 (1983); Davis, J. Wood Chem. Technol. 18: 235-252 (1988).

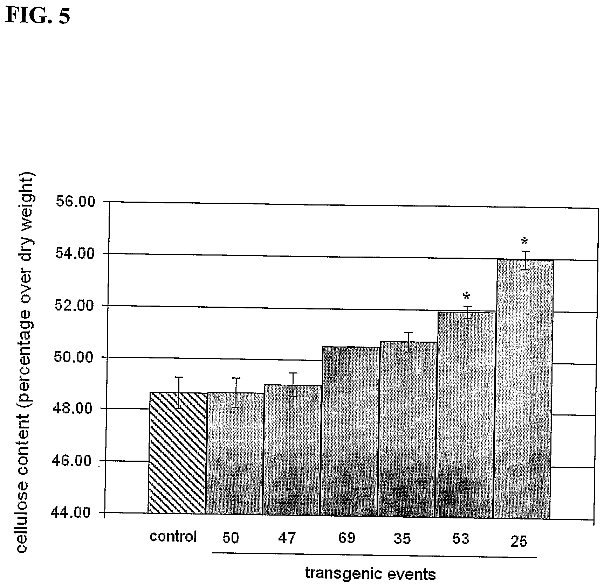

Two of the transgenic events, known to express the transgene according to procedure detailed in Example 3, showed a statistically significant increase in cellulose content (FIG. 5). Transgenic event 25 exhibits 54% cellulose as compared to 48.6% in control plants, representing a significant increase of 11% in cellulose content (P<0.01, t-test). Transgenic event 53 exhibits 52% cellulose as compared to 48.6% in control plants, representing 7% increase in cellulose content (FIG. 5; P<0.01, t-test). Additionally, transgenic line 25 exhibited a marked decrease in lignin content (19, 15% as compared to 23.3% in control plants; P<0.01, t-test), as assayed by the Klason gravimetric method.

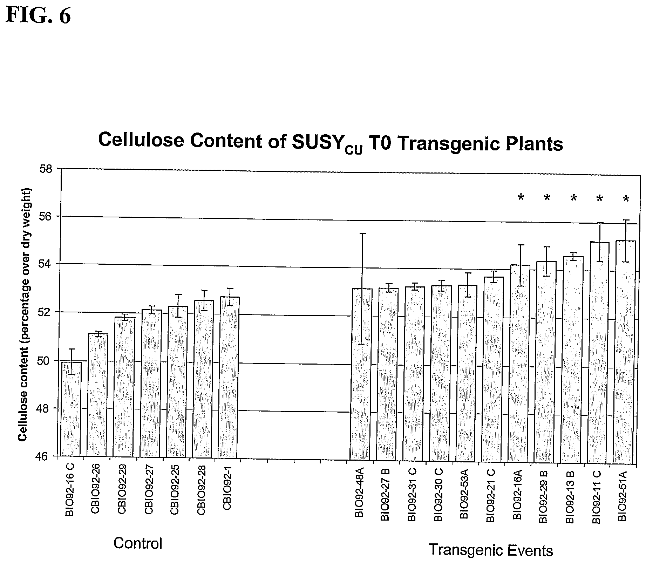

Five of the eleven T0 transgenic events, transformed with the construct comprising a modified codon usage-adapted sucrose synthase gene from *Anabaena* sp., showed a statistically significant increase in cellulose content. Transgenic events BIO92-51A, BIO92-11C, BIO92-13B, BIO92-29B and BIO92-16A exhibit a significant increase in cellulose content when compared to the mean value of control plants (P<0.05, t-test) (FIG.6). For example, transgenic event BIO92-51A exhibits 55.28% cellulose as compared to 51.7% in control non-transgenic plants, representing a significant increase of ≈7% in cellulose content.

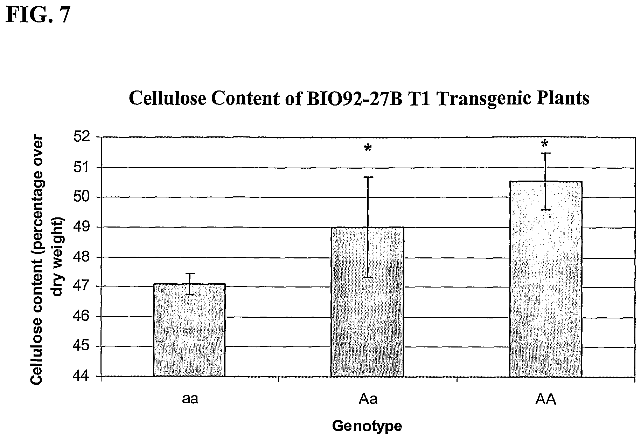

After grown to maturity, the T0 events from the codon usage-adapted sucrose synthase gene were selfed to generate T1 lines. Plants that are homozygote dominant present a significant increase of 7% in cellulose content (P<0.05, t-test), when compared to homozygote recessive plants (FIG.7).

## Claims

1. A transgenic plant comprising a codon-optimized non-plant sucrose synthase nucleic acid molecule operably linked to an vascular system-specific promoter, wherein said plant has increased cellulose content compared with a non-transgenic plant lacking said molecule.

2. The transgenic plant of claim 1, obtained by transforming a plant with an expression vector comprising said codon-optimized non-plant sucrose synthase nucleic acid molecule, under the control of a vascular system-specific promoter capable of functioning in a plant.

3. The transgenic plant of claim 1, wherein said nucleic acid molecule is a nucleotide sequence comprising (a) SEQ ID NO.: 2 or (b) SEQ ID NO: 2 modified such that the modification does not result in the loss of sucrose synthase enzyme activity compared with a non-transgenic plant lacking said nucleotide sequence.

4. The transgenic plant of claim 1, wherein said plant is a dicotyledon.

5. The transgenic plant of claim 1, wherein said plant is a monocotyledon.

6. The transgenic plant of claim 1, wherein said plant is a gymnosperm.

7. The transgenic plant of claim 1, wherein said plant is a woody tree.

8. The transgenic plant of claim 1, wherein said plant is a *Eucalyptus* plant.

9. The transgenic plant of claim 1, wherein said plant is a *Populus* plant.

10. The transgenic plant of claim 1, wherein said plant is a *Pinus* plant.

11. A method for producing a transgenic plant with increased cellulose content as compared to a non-transgenic plant, comprising (i) transforming a plant cell with a codon-optimized non-plant sucrose synthase sequence under the control of a vascular system-specific promoter capable of functioning in a plant, (ii) culturing said transformed plant cell under conditions that promote growth of a plant, and (iii) selecting a transgenic plant that exhibits increased cellulose content.

12. The method of claim 11, wherein the nucleotide sequence is a sucrose synthase gene comprising a nucleotide sequence comprising (a) SEQ ID NO: 2 or (b) SEQ ID NO: 2 modified such that the modification does not result in the loss of sucrose synthase enzyme activity compared with a non-transgenic plant lacking said nucleotide sequence.

13. The method of claim 12, wherein said transgenic plant is a dicotyledon.

14. The method of claim 12, wherein said transgenic plant is a monocotyledon.

15. The method of claim 12, wherein said transgenic plant is a gymnosperm.

## Patentansprüche

1. Transgene Pflanze, umfassend ein codonoptimiertes nichtpflanzliches Saccharosesynthase-Nukleinsäuremolekül in operativer Verknüpfung mit einem gefäßsystemspezifischen Promotor, wobei die Pflanze im Vergleich zu einer nicht transgenen Pflanze ohne dieses Molekül einen erhöhten Cellulosegehalt aufweist.

2. Transgene Pflanze nach Anspruch 1, erhalten dadurch, dass man eine Pflanze mit einem Expressionsvektor umfassend das codonoptimierte nichtpflanzliche Saccharosesynthase-Nukleinsäuremolekül unter der Kontrolle eines gefäßsystemspezifischen Promotors, der fähig ist, in einer Pflanze zu funktionieren, transformiert.

3. Transgene Pflanze nach Anspruch 1, wobei es sich bei dem Nukleinsäuremolekül um einen Nukleotidsequenz umfassend (a) SEQ ID NO: 2 oder (b) SEQ ID NO: 2 handelt, die so modifiziert ist, dass die Modifikation im Vergleich zu einer nicht transgenen Pflanze ohne diese Nukleotidsequenz nicht zum Verlust von Saccharosesynthase-Enzymaktivität führt.

4. Transgene Pflanze nach Anspruch 1, wobei es sich bei der Pflanze um eine Dikotyledone handelt.

5. Transgene Pflanze nach Anspruch 1, wobei es sich bei der Pflanze um eine Monokotyledone handelt.

6. Transgene Pflanze nach Anspruch 1, wobei es sich bei der Pflanze um eine Gymnosperme handelt.

7. Transgene Pflanze nach Anspruch 1, wobei es sich bei der Pflanze um einen Holzbaum handelt.

8. Transgene Pflanze nach Anspruch 1, wobei es sich bei der Pflanze um eine *Eukalyptus*-Pflanze handelt.

9. Transgene Pflanze nach Anspruch 1, wobei es sich bei der Pflanze um eine *Populus*-Pflanze handelt.

10. Transgene Pflanze nach Anspruch 1, wobei es sich bei der Pflanze um eine *Pinus*-Pflanze handelt.

11. Verfahren zum Erzeugen einer transgenen Pflanze mit erhöhtem Cellulosegehalt im Vergleich zu einer nicht transgenen Pflanze, umfassend (i) das Transformieren einer Pflanzenzelle mit einer codonoptimierten nicht pflanzlichen Saccharosesynthasesequenz unter der Kontrolle eines gefäßsystemspezifischen Promotors, der fähig ist, in einer Pflanze zu funktionieren, (ii) das Kultivieren der transformierten Pflanzenzelle unter Bedingungen, die das Wachstum einer Pflanze fördern, und (iii) das Selektieren einer transgenen Pflanze, die einen erhöhten Cellulosegehalt aufweist.

12. Verfahren nach Anspruch 11, wobei es sich bei der Nukleotidsequenz um ein Saccharosesynthasegen umfassend eine Nukleotidsequenz umfassend (a) SEQ ID NO: 2 oder (b) SEQ ID NO: 2 handelt, die so modifiziert ist, dass die Modifikation nicht zum Verlust der Saccharosesynthase-Enzymaktivität im Vergleich zu einer nicht transgenen Pflanze ohne diese Nukleotidsequenz führt.

13. Verfahren nach Anspruch 12, wobei es sich bei der transgenen Pflanze um eine Dikotyledone handelt.

14. Verfahren nach Anspruch 12, wobei es sich bei der transgenen Pflanze um eine Monokotyledone handelt.

15. Verfahren nach Anspruch 12, wobei es sich bei der transgenen Pflanze um eine Gymnosperme handelt.

## Revendications

1. Plante transgénique comprenant une molécule d'acide nucléique de saccharose synthase non végétale à codons optimisés, liée de manière opérante à un promoteur spécifique du système vasculaire, où ladite plante possède une teneur accrue en cellulose par rapport à une plante non transgénique dépourvue de ladite molécule.

2. Plante transgénique selon la revendication 1, obtenue par la transformation d'une plante par un vecteur d'expression comprenant ladite molécule d'acide nucléique de saccharose synthase non végétale à codons optimisés, sous le contrôle d'un promoteur spécifique du système vasculaire capable de fonctionner dans une plante.

3. Plante transgénique selon la revendication 1, où ladite molécule d'acide nucléique est une séquence nucléotidique comprenant (a) SEQ ID n° 2 ou (b) SEQ ID n° 2 modifiée de telle sorte que la modification n'entraîne pas la perte de l'activité de l'enzyme saccharose synthase par rapport à une plante non transgénique dépourvue de ladite séquence nucléotidique.

4. Plante transgénique selon la revendication 1, où ladite plante est une dicotylédone.

5. Plante transgénique selon la revendication 1, où ladite plante est une monocotylédone.

6. Plante transgénique selon la revendication 1, où ladite plante est une gymnosperme.

7. Plante transgénique selon la revendication 1, où ladite plante est un arbre ligneux.

8. Plante transgénique selon la revendication 1, où ladite plante est une plante d'*Eucalyptus.*

9. Plante transgénique selon la revendication 1, où ladite plante est une plante de *Populus.*

10. Plante transgénique selon la revendication 1, où ladite plante est une plante de *Pinus.*

11. Méthode de production d'une plante transgénique ayant une teneur accrue en cellulose par rapport à une plante non transgénique, comprenant (i) la transformation d'une cellule végétale par une séquence de saccharose synthase non végétale à codons optimisés, sous le contrôle d'un promoteur spécifique du système vasculaire capable de fonctionner dans une plante, (ii) la culture de ladite cellule végétale transformée dans des conditions qui favorisent la croissance d'une plante, et (iii) la sélection d'une plante transgénique qui présente une teneur accrue en cellulose.

12. Méthode selon la revendication 11, où la séquence nucléotidique est un gène de saccharose synthase comprenant une séquence nucléotidique comprenant (a) SEQ ID n° 2 ou (b) SEQ ID n° 2 modifiée de telle sorte que la modification n'entraîne pas la perte de l'activité de l'enzyme saccharose synthase par rapport à une plante non transgénique dépourvue de ladite séquence nucléotidique.

13. Méthode selon la revendication 12, où ladite plante transgénique est une dicotylédone.

14. Méthode selon la revendication 12, où ladite plante transgénique est une monocotylédone.

15. Méthode selon la revendication 12, où ladite plante transgénique est une gymnosperme.


### SEQUENCE LISTING

<110> PAPES, Fabio
   ARRUDA, Paulo
<120> Polynucleotides, DNA constructs and methods for the alteration of plant cellulose
   content
<140>
   <141>
<150> US 60/728,743
   <151> 2005-10-21
<160> 8
<210> 1
   <211> 2429
   <212> DNA
   <213> Anabaena sp. PCC 7120
<220>
   <221> DNA
   <222> (1)...(2429)
   <223> Anabaena sucrose synthase, genomic clone
<400> SEQ ID NO: 1
<210> 2
   <211> 2430
   <212> DNA
   <213> Anabaena sp. PCC 7120
<220>
   <221> synthetic
   <222> (1)...(2430)
   <223> synthetic codon usage-adapted Anabaena sucrose synthase
<400> 2
   catatgtctg agctcatgca ggctatcctc gattctgagg agaagcacga tctcaggggc   60
   ttcatctctg agctcaggca gcaggataag aactacctcc tcaggaacga tatcctcaac  120
   gtgtacgctg agtactgctc taagtgccag aagcctgaga cctcttacaa gttctctaac  180
   ctctctaagc tcatctacta cacccaggag atcatccagg aggattctaa cttctgcttc  240
   atcatcaggc ctaagatcgc tgctcaggag gtgtacaggc tcaccgctga tctcgatgtg  300
   gagcctatga ccgtgcagga gctcctcgat ctcagggata ggctcgtgaa caagttccac  360
   ccttacgagg gcgatatcct cgagctcgat ttcggccctt tctacgatta cacccctacc  420
   atcagggatc ctaagaacat cggcaagggc gtgcagtacc tcaacaggta cctctcttct  480
   aagctcttcc aggattctca gcagtggctc gagtctctct tcaacttcct caggctccac  540
   aactacaacg gcatccagct cctcatcaac caccagatcc agtctcagca gcagctctct  600
   cagcaggtga agaacgctct caacttcgtg tctgataggc ctaacgatga gccttacgag  660
   cagttcaggc tccagctcca gaccatgggc ttcgagcctg gctggggcaa caccgcttct   720
   agggtgaggg ataccctcaa catcctcgat gagctcatcg attctcctga tcctcagacc   780
   ctcgaggctt tcatctctag gatccctatg atcttcagga tcgtgctcgt gtctgctcac   840
   ggctggttcg gccaggaggg cgtgctcggc aggcctgata ccggcggcca ggtggtgtac   900
   gtgctcgatc aggctaagaa cctcgagaag cagctccagg aggatgctat cctcgctggc   960
   ctcgaggtgc tcaacgtgca gcctaaggtg atcatcctca ccaggctcat ccctaactct  1020
   gatggcaccc tctgcaacca gaggctcgag aaggtgtacg gcaccgagaa cgcttggatc  1080
   ctcagggtgc ctctcaggga gttcaaccct aagatgaccc agaactggat ctctaggttc  1140
   gagttctggc cttacctcga gaccttcgct atcgattctg agagggagct cctcgctgag  1200
   ttccagggca ggcctgatct catcgtgggc aactacaccg atggcaacct cgtggctttc  1260
   ctcctcacca ggaggatgaa ggtgacccag tgcaacatcg ctcacgctct cgagaagtct  1320
   aagtacctct tctctaacct ctactggcag gatctcgagg agaagtacca cttctctctc  1380
   cagttcaccg ctgatctcat cgctatgaac gctgctaact tcgtgatctc ttctacctac  1440
   caggagatcg tgggcacccc tgattctatc ggccagtacg agtcttacaa gtgcttcacc  1500
   atgcctgagc tctaccacgt ggtgaacggc atcgagctct tctctcctaa gttcaacgtg  1560
   gtgcctcctg gcgtgaacga gaactcttac ttcccttaca cccagaccca gaacaggatc  1620
   gagtctgata gggataggct cgaggagatg ctcttcaccc tcgaggattc ttctcagatc  1680
   ttcggcaagc tcgatgatcc taacaagagg cctatcttct ctatggctag gctcgatagg  1740
   atcaagaacc tcaccggcct cgctgagtgc ttcggccagt ctcaggagct ccaggagagg  1800
   tgcaacctca tcctcgtggc tggcaagctc aggatcgagg agtctgagga taacgaggag  1860
   aaggatgaga tcgtgaagct ctacaggatc atcgatgagt acaacctcca cggcaagatc  1920
   aggtggctcg gcgtgaggct ctctaagaac gattctggcg agatctacag ggtgatctgc  1980
   gataggcagg gcatcttcgt gcagcctgct ctcttcgagg ctttcggcct caccatcctc  2040
   gagtctatga tctctggcct ccctaccttc gctacccagt tcggcggccc tctcgagatc  2100
   atccaggata agatcaacgg cttctacatc aaccctaccc acctcgagga gaccgctacc  2160
   aagatcctcg atttcgtgac caagtgcgag cagaacccta actactggaa catcatctct  2220
   gagaaggcta tcgatagggt gtactctacc tacacctgga agatccacac caccaagctc  2280
   ctcaccctcg ctaggatcta cggcttctgg aacttcacct ctaaggagaa gagggaggat  2340
   ctcctcaggt acctcgagtc tctcttctac ctcatctaca agcctagggc tcagcagctc  2400
   ctcgagcagc acaagtacag gtgatctaga                                   2430
<210> 3
   <211> 806
   <212> PRT
   <213> Anabaena sp. PCC 7120
<400> 3
   Met Ser Glu Leu Met Gln Ala Ile Leu Asp Ser Glu Glu Lys His Asp
                   5                   10                  15
   Leu Arg Gly Phe Ile Ser Glu Leu Arg Gln Gln Asp Lys Asn Tyr Leu
               20                  25                  30
   Leu Arg Asn Asp Ile Leu Asn Val Tyr Ala Glu Tyr Cys Ser Lys Cys
           35                  40                  45
   Gln Lys Pro Glu Thr Ser Tyr Lys Phe Ser Asn Leu Ser Lys Leu Ile
       50                  55                  60
   Tyr Tyr Thr Gln Glu Ile Ile Gln Glu Asp Ser Asn Phe Cys Phe Ile
   65                  70                  75                  80
   Ile Arg Pro Lys Ile Ala Ala Gln Glu Val Tyr Arg Leu Thr Ala Asp
                   85                  90                  95
   Leu Asp Val Glu Pro Met Thr Val Gln Glu Leu Leu Asp Leu Arg Asp
               100                 105                 110
   Arg Leu Val Asn Lys Phe His Pro Tyr Glu Gly Asp Ile Leu Glu Leu
           115                 120                 125
   Asp Phe Gly Pro Phe Tyr Asp Tyr Thr Pro Thr Ile Arg Asp Pro Lys
       130                 135                 140
   Asn Ile Gly Lys Gly Val Gln Tyr Leu Asn Arg Tyr Leu Ser Ser Lys
   145                 150                 155                 160
   Leu Phe Gln Asp Ser Gln Gln Trp Leu Glu Ser Leu Phe Asn Phe Leu
                   165                 170                 175
   Arg Leu His Asn Tyr Asn Gly Ile Gln Leu Leu Ile Asn His Gln Ile
               180                 185                 190
   Gln Ser Gln Gln Gln Leu Ser Gln Gln Val Lys Asn Ala Leu Asn Phe
           195                 200                 205
   Val Ser Asp Arg Pro Asn Asp Glu Pro Tyr Glu Gln Phe Arg Leu Gln
       210                 215                 220
   Leu Gln Thr Met Gly Phe Glu Pro Gly Trp Gly Asn Thr Ala Ser Arg
   225                 230                 235                 240
   Val Arg Asp Thr Leu Asn Ile Leu Asp Glu Leu Ile Asp Ser Pro Asp
                   245                 250                 255
   Pro Gln Thr Leu Glu Ala Phe Ile Ser Arg Ile Pro Met Ile Phe Arg
               260                 265                 270
   Ile Val Leu Val Ser Ala His Gly Trp Phe Gly Gln Glu Gly Val Leu
           275                 280                 285
   Gly Arg Pro Asp Thr Gly Gly Gln Val Val Tyr Val Leu Asp Gln Ala
       290                 295                 300
   Lys Asn Leu Glu Lys Gln Leu Gln Glu Asp Ala Ile Leu Ala Gly Leu
   305                 310                 315                 320
   Glu Val Leu Asn Val Gln Pro Lys Val Ile Ile Leu Thr Arg Leu Ile
                   325                 330                 335
   Pro Asn Ser Asp Gly Thr Leu Cys Asn Gln Arg Leu Glu Lys Val Tyr
               340                 345                 350
   Gly Thr Glu Asn Ala Trp Ile Leu Arg Val Pro Leu Arg Glu Phe Asn
           355                 360                 365
   Pro Lys Met Thr Gln Asn Trp Ile Ser Arg Phe Glu Phe Trp Pro Tyr
       370                 375                 380
   Leu Glu Thr Phe Ala Ile Asp Ser Glu Arg Glu Leu Leu Ala Glu Phe
   385                 390                 395                 400
   Gln Gly Arg Pro Asp Leu Ile Val Gly Asn Tyr Thr Asp Gly Asn Leu
                   405                 410                 415
   Val Ala Phe Leu Leu Thr Arg Arg Met Lys Val Thr Gln Cys Asn Ile
               420                 425                 430
   Ala His Ala Leu Glu Lys Ser Lys Tyr Leu Phe Ser Asn Leu Tyr Trp
           435                 440                 445
   Gln Asp Leu Glu Glu Lys Tyr His Phe Ser Leu Gln Phe Thr Ala Asp
       450                 455                 460
   Leu Ile Ala Met Asn Ala Ala Asn Phe Val Ile Ser Ser Thr Tyr Gln
   465                 470                 475                 480
   Glu Ile Val Gly Thr Pro Asp Ser Ile Gly Gln Tyr Glu Ser Tyr Lys
                   485                 490                 495
   Cys Phe Thr Met Pro Glu Leu Tyr His Val Val Asn Gly Ile Glu Leu
               500                 505                 510
   Phe Ser Pro Lys Phe Asn Val Val Pro Pro Gly Val Asn Glu Asn Ser
           515                 520                 525
   Tyr Phe Pro Tyr Thr Gln Thr Gln Asn Arg Ile Glu Ser Asp Arg Asp
       530                 535                 540
   Arg Leu Glu Glu Met Leu Phe Thr Leu Glu Asp Ser Ser Gln Ile Phe
   545                 550                 555                 560
   Gly Lys Leu Asp Asp Pro Asn Lys Arg Pro Ile Phe Ser Met Ala Arg
                   565                 570                 575
   Leu Asp Arg Ile Lys Asn Leu Thr Gly Leu Ala Glu Cys Phe Gly Gln
               580                 585                 590
   Ser Gln Glu Leu Gln Glu Arg Cys Asn Leu Ile Leu Val Ala Gly Lys
           595                 600                 605
   Leu Arg Ile Glu Glu Ser Glu Asp Asn Glu Glu Lys Asp Glu Ile Val
       610                 615                 620
   Lys Leu Tyr Arg Ile Ile Asp Glu Tyr Asn Leu His Gly Lys Ile Arg
   625                 630                 635                 640
   Trp Leu Gly Val Arg Leu Ser Lys Asn Asp Ser Gly Glu Ile Tyr Arg
                   645                 650                 655
   Val Ile Cys Asp Arg Gln Gly Ile Phe Val Gln Pro Ala Leu Phe Glu
               660                 665                 670
   Ala Phe Gly Leu Thr Ile Leu Glu Ser Met Ile Ser Gly Leu Pro Thr
           675                 680                 685
   Phe Ala Thr Gln Phe Gly Gly Pro Leu Glu Ile Ile Gln Asp Lys Ile
       690                 695                 700
   Asn Gly Phe Tyr Ile Asn Pro Thr His Leu Glu Glu Thr Ala Thr Lys
   705                 710                 715                 720
   Ile Leu Asp Phe Val Thr Lys Cys Glu Gln Asn Pro Asn Tyr Trp Asn
                   725                 730                 735
   Ile Ile Ser Glu Lys Ala Ile Asp Arg Val Tyr Ser Thr Tyr Thr Trp
               740                 745                 750
   Lys Ile His Thr Thr Lys Leu Leu Thr Leu Ala Arg Ile Tyr Gly Phe
               755                 760                 765
   Trp Asn Phe Thr Ser Lys Glu Lys Arg Glu Asp Leu Leu Arg Tyr Leu
       770                 775                 780
   Glu Ser Leu Phe Tyr Leu Ile Tyr Lys Pro Arg Ala Gln Gln Leu Leu
   785                 790                 795                 800
   Glu Gln His Lys Tyr Arg
                   805
<210> 4
   <211> 27
   <212> DNA
   <213> Artificial sequence
<220>
   <223> Synthetic primer/oligonucleotide Susy_s3
<400> 4
   atccatatgt cagaattgat gcaagcg 27
<210> 5
   <211> 33
   <212> DNA
   <213> Artificial sequence
<220>
   <223> Synthetic primer/oligonucleotide Susy_as3
<400> 5
   tcagatctta ccgatattta tgctgttcta ata 33
<210> 6
   <211> 20
   <212> DNA
   <213> Artificial sequence
<220>
   <223> Susy_seq3 primer
<400> 6
   caagaattgc aagaacgttg 20
<210> 7
   <211> 28
   <212> DNA
   <213> Artificial sequence
<220>
   <223> Synthetic primer/oligonucleotide SUSY_CU_RT1
<400> 7
   ctaggctcga taggatcaag aacctcac 28
<210> 8
   <211> 28
   <212> DNA
   <213> Artificial sequence
<220>
   <223> Synthetic primer/oligonucleotide SUSY_CU_RT2
<400> 8
   gatagccttc tcagagatga tgttccag 28